# EUROPEAN PATENT SPECIFICATION

(11) **EP 0 228 375 B1**
(45) Date of publication and mention of the grant of the patent: **16.06.1993**
(21) Application number: 85905130.2
(22) Date of filing: 07.10.1985
(51) Int. Cl.: A61B 1/22

(54) **AN INTERCHANGEABLE FUNNEL FOR ATTACHMENT TO INSTRUMENTS USED FOR THE DIAGNOSTIC AND TREATMENT OF OPENINGS TO BODY CANALS**
AUSWECHSELBARER TRICHTERFÖRMIGER ZUSATZ FÜR INSTRUMENTE ZUR DIAGNOSTIK UND BEHANDLUNG VON VON AUSSEN ZUGÄNGLICHEN KÖRPERHÖHLEN
ENTONNOIR INTERCHANGEABLE SE FIXANT A DES INSTRUMENTS UTILISES POUR LE DIAGNOSTIC ET LE TRAITEMENT D'OUVERTURES DE CANAUX DU CORPS HUMAIN

(30) Priority: 08.10.1984 SE 8405004
(43) Date of publication of application: 15.07.1987
(73) Proprietor: THOREN, Jörgen, S-502 60 Boras (SE)
(72) Inventor: THOREN, Jörgen, S-502 60 Boras (SE)
(74) Representative: Mossmark, Anders
(86) International application number: SE8500384
(87) International publication number: WO8601992

(56) References cited:
- DE-A- 2 029 892
- DE-A- 2 403 882
- US-A- 4 006 738

## Description

### Technical field:

The present invention relates to an interchangeable funnel for attachment to instruments for the diagnosis and treatment of openings to body canals.

### Background:

For the purpose of diagnosing and treating the ears, for example, use is made of an ophtalmoscope, an instrument which is also known as an otoscope, to the head of which a disposable funnel is fitted. These heads are available in several different models which incorporate various types of means for locking the funnel securely in position. A previously disclosed funnel is secured to the head by means of a snap-in lock, (see e.g. DE-A-2 403 882), whilst a second type of funnel is secured by causing a bead arranged on the funnel to rotate in a groove in a funnel-shaped part on the head (see e.g. DE-A-2 029 892). This means that different funnels are required for different types of head. The object of the present invention is to make available a funnel for the diagnosis and treatment of openings to body canals which can be combined with different types of head.

### The solution:

The aforementionned object is achieved by means of a funnel in accordance with claim 1.

### Brief description of drawings:

The invention is described below in greater detail as an illustrative embodiment with reference to the accompanying drawings, of which Fig. 1 shows a side view of a funnel in accordance with the invention, Fig. 2 shows a view of the funnel at an angle from the front, Fig. 3 shows a section III-III through the funnel, Fig. 4 shows on an enlarged scale a partially cut-away section III-III through the edge part of the funnel, Fig. 5 shows on an enlarged scale a partially cut-away section III-III through the edge part of the funnel in accordance with a second embodiment, Fig. 6 shows an otoscope with a section of a head intended for a funnel with rotating locking, Fig. 7 shows a view from above of a head with rotating locking, and Fig. 8 shows a side view of a head with snap-in locking in which the funnel is indicated by dotted and dashed lines.

### Best mode of carrying out the invention:

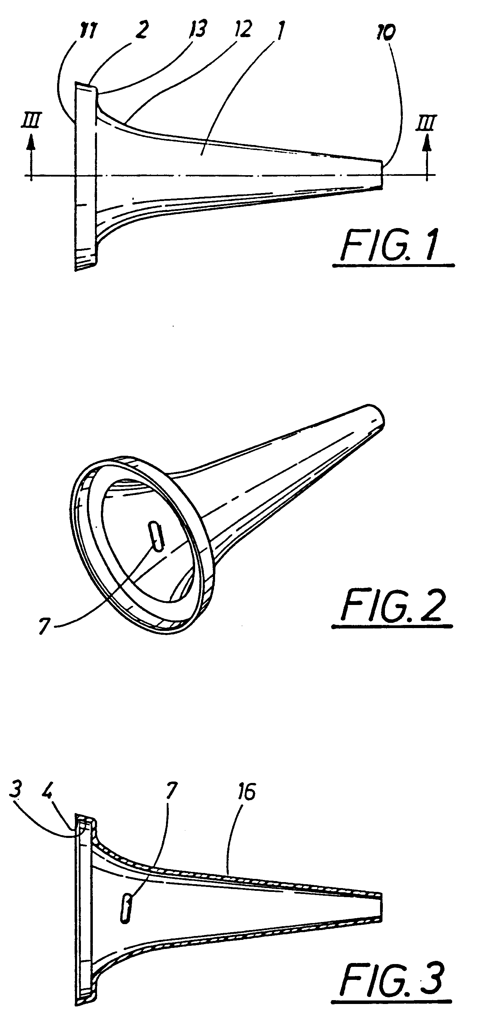

As will be appreciated from the example illustrated in Figs. 1-4, the funnel, referred to below as the combination funnel, exhibits an outwardly smooth, conical body 1 which is terminated at its broader end by a straight edge 2 and is open at both ends. The inside 3 of the edge 2 is terminated by a peripheral bead 4 so arranged as to snap into engagement in a concave groove 25 on a head 6 of an ophthalomoscope or otoscope, that is to say an instrument for the diagnosis and treatment of canals in the ear and nose or similar. A further bead 7 is so arranged on the inside of the tapering part of the funnel as to form an angle in relation to the line of the edge and is executed to fit in a groove 8 on a second head 9 of an otoscope of a different design, which is described in greater detail below. As has already been stated, the funnel exhibits a small opening 10 at the front and a larger opening 11 at the rear. The front opening 10 is designed to be introduced into the actual body canal, whilst the rear opening is designed to face towards the head 6 or 9 of the instrument. The funnel exhibits strictly conical form from the geometrical point of view at the front for a certain distance ahead of the bead 7, when viewed from the front opening 10 whereupon the funnel changes to exhibit a progressively increasing cross-sectional area by following a curved contour line 12 so as to change to a radial and essentially flat annular surface 13 in conjunction with the edge part 2. The inside 3 of the edge part 2 in the example illustrated exhibits the form of the generating surface of a truncated cone facing in the same direction as the cone constituted by the funnel as a whole, whereby the funnel exhibits an increasing diameter in a direction from its smaller end 10 as far as the peripheral bead 4, where the internal diameter again reduces over a short distance. With the exception of the peripheral bead 4 the funnel exhibits to all intents and purposes a uniform thickness of material, so that the contour form of the funnel has the same curvature both on its inside and on its outside, but with the bead 7 representing an exception to this.

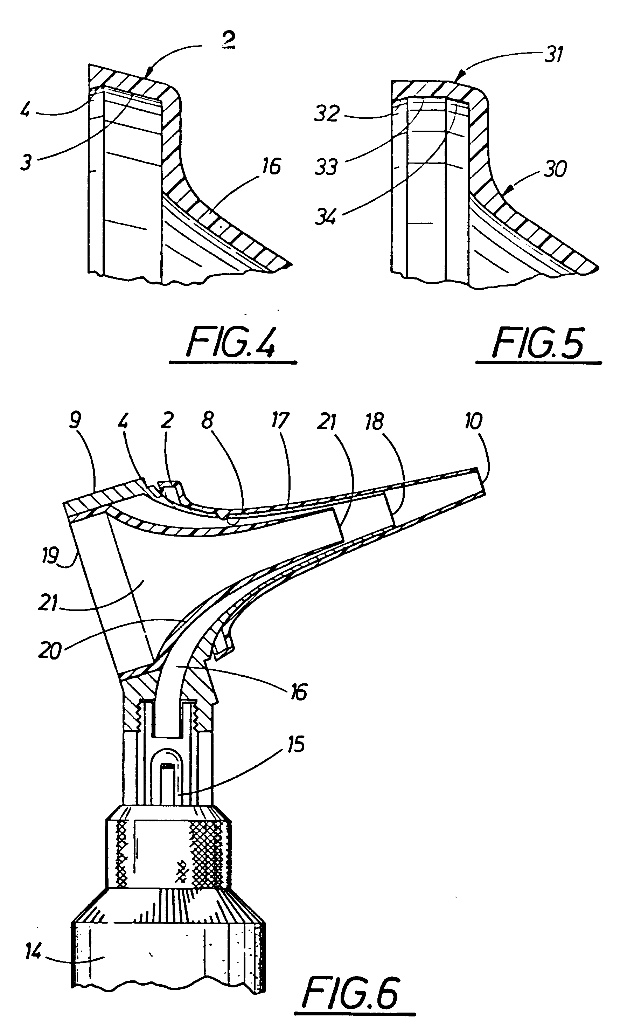

Shown in Fig. 6 as a partially cut-away view is the otoscope with the head 9 for rotating locking of the funnel. The head is attached to a handle component 14 which serves at the same time as a battery holder for a battery for powering a light source 15 in the form of an incandescent lamp situated at the upper end of the holder. The head is screwed onto the handle component and contains a fibre optics system 16 which conducts the light from the light source in the direction of the front opening 10 of the funnel. The head 9 of the model illustrated in Fig. 6 exhibits a funnel-shaped component 17 with a front opening 18 and a considerably larger rear opening 19. Internally the head similarly exhibits a funnel-shaped component 20, the front opening 21 of which is situated inside the opening 18, whilst the rear opening 19 is common to the two funnel-shaped components. There extends between the openings 18 and 19 a transcurrent hole 21, through which diagnosis and treatment are performed.

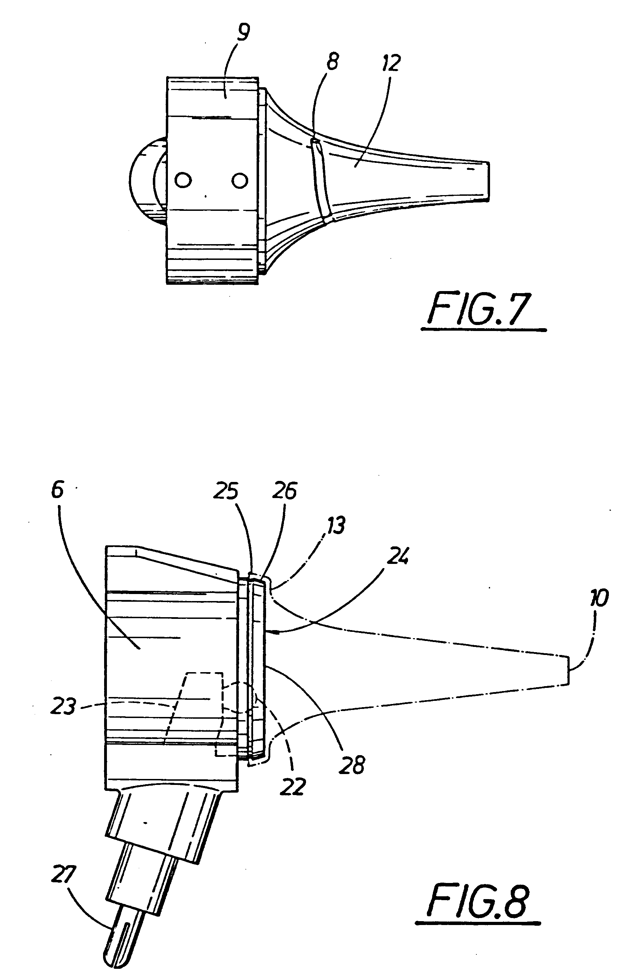

Shown in Fig. 8 is the head 6 for the snap-in locking of the funnel in accordance with the invention. This head has no funnel-shaped component, but is executed essentially in the form of an annular flange with a transcurrent hole, inside which a light source in the form of an incandescent lamp 22 is attached in a lamp holder 23 which is situated inside the transcurrent hole. The front, annular flange part 24 of the head 6 constitutes a means of attachment for the funnel. The flange part 24 exhibits an annular, for example concave, groove 25 intended to interact with the peripheral bead 4 on the funnel. The flange part 24 exhibits outside the groove 25 an annular part 26 in the form of the outer surface of a cone, which corresponds in its form and dimensions to the inside 3 of the edge part of the funnel. The handle component is not shown in Fig. 8 for the sake of simplicity, although this may exhibit the same external appearance as the handle component in accordance with Fig. 6, although the latter does not contain any light source, but simply an electrical connection device designed to fit the electrical connection pin 27 via which the power supply to the light source 22 is provided.

A second embodiment of the combination funnel is shown in Fig. 5, which also exhibits a smooth, conical body 30 which is terminated at its wider end by a slightly curved edge part 31 and is open at both ends. The edge part is terminated by a straight edge which is also executed on the inside in the form of a bead 32 so arranged as to provide a snap fit in the concave groove 25 in the head 6 of the ophthalmoscope or otoscope. The inside of the edge part exhibits a straight cylindrical generating surface 33 relative to the straight edge part of the opening and a generating surface 34 with the form of a truncated cone set at an angle to the latter generating surface. In other respects this funnel is executed in the same fashion as the combination funnel previously referred to.

By executing the funnel in the fashion described above, it is thus possible for one and the same funnel to be used for two different types of heads for the otoscope instrument. Where the instrument in accordance with Figs. 6 and 7 is used with a funnel-shaped component 17, the interchangeable funnel, which in practice is a disposable funnel which is used once only, is pushed onto the funnel-shaped part of the head whilst being rotated, causing locking to occur as the bead 7 on the inside of the funnel is introduced into the angled groove 8 on the funnel-shaped component 17 of the head. By executing the groove at an angle, the bead 7 acts in conjunction with the groove 8 in the same way as the interaction between the threads on a bolt and a nut, which means that the funnel is pushed further in on the funnel-shaped part of the head until the funnel, as may be appreciated from Fig. 6, makes contact with its inside against the outside of the funnel-shaped part. As will be appreciated from this Figure, the edge part 2 of the funnel is not used in this instance, but is in no sense in the way because the edge part 2 does not come into contact with any part of the head 9. The peripheral bead 4 may possibly come into contact with the surface of the funnel-shaped part 17, which only produces a stabilizing effect for the funnel. The funnel thus acts as a hygienic barrier which is replaced on each occasion before examining a patient's auditory canal, nasal canal or similar, in conjunction with which the canal is illuminated by the directional beam of light from the light source 15 via the fibre optics system 16. The illuminated area can be studied freely by the doctor looking at the area concerned through the opening 19. For the sake of good order it must be pointed out that the funnel is also known as the speculum.

One and the same funnel can thus find an application also when using an otoscope instrument with a head 6 of the type illustrated in Fig. 8. In this case neither the conical part of the funnel nor the part with a curved contour is used as the supporting surface against any funnel-shaped part of the head, with attachment instead taking place only with the flanged part 24, for which purpose use is made of the edge part 2 of the funnel. All that is required in this case is simply for the edge part 2 of the funnel to be pushed over the flange part 24 of the head 6, so that the peripheral bead 4 snaps into engagement with the annular groove 25 so as to provide support on the one hand with the outside 3 of the edge part 2 in the form of the outer surface of a cone, and on the other hand with the surface 13 which extends in the radial plane and which makes contact with a forward-facing edge 28 of the flange part 24. Very stable and reliable attachment is achieved in this way, without the other bead 7 being utilized for locking. In this case, too, the body canal in question is illuminated by directing a beam of light through the funnel and out via its front end 10 from the incandescent lamp 22, in conjunction with which the illuminated area is observed by the doctor through the transcurrent hole in the head 6 and through the similarly transcurrent hole in the funnel.

By executing the inside of the edge part 31 with two generating surfaces set at an angle relative to one another, the grip on the flange part 24 is increased, when the force generated essentially by the snap-in effect is directed essentially at right-angles to the aforementioned flange part. Furthermore the straight cylindrical generating surface 33 permits greater adaptability to the different types of flange parts which may be encountered on variants of the head 6, so that the entire area of the generated surface can be utilized to provide contact with the flange part without affecting the grip of the funnel on the flange part. The contact between the generating surface and the aforementioned flange part is thus restricted on one side by the peripheral bead 32 and on the other side by the angled generating surface 34.

The funnel is preferably executed in a plastics material having appropriately dimensioned flexibility such that the peripheral bead 4, for example, is flexible yet provides a distinct snap-in engagement position. Similarly, the bead 7 for the attachment groove 8 should be permitted by the general elastic flexibility of the funnel to flex and to snap into engagement in the groove 1. The funnel should preferably be executed in polypropylene, which possesses a low coefficient of friction; this material, when injection moulded, produces very few burrs. This latter characteristic is significant, since burrs give rise to reflections inside the funnel which impair the quality of the view through the funnel. Another important factor with regard to the elimination of the risk of burrs being formed in the course of the injection moulding process is the special method of injection moulding which is used in this case, in conjunction with which an injection moulding tool with special parting of the mould and with vent ducts is used. It is also advantageous to execute the funnel with a glossy outside surface which feels comfortable to the skin, and with a matt inside surface which further reduces the risk of reflections on the inside. Polypropylene is a material which also tolerates high temperatures and which offers particular advantages in view of the fact that the funnel is accordingly able to be sterilized after it has been injection moulded.

The invention is not restricted to the illustrative embodiment described above and portrayed in the drawings. The funnel can be executed in different lengths to suit different applications. A long funnel, for example, will thus eliminate the risk of wax from the ear finding its way into the instrument.

## Claims

1. A funnel for attachment to medical instruments used for the diagnosis and treatment of openings to body canals so constructed as to be attachable to a head (6, 9) of the instrument of at least two different types, of which one head (6) exhibits an annular flanged part (24) and the other head (9) exhibits a funnel-shaped part (12) with a groove (8), which funnel exhibits a small front opening (10) and a larger rear opening (11) together with a transcurrent hole between them, wherein the funnel is provided at its larger end (11) with an edge part (2) extending around the opening so arranged as to engage by means of a snap-in lock around the flange part (24) on the first head (6), and in that the inside of the funnel is provided with a bead (7) so arranged as to fit into the groove (8) on the second head (9), whereby the funnel is able as a single design to be used for both the aforementioned types of head.

2. A funnel according to Patent Claim 1, **characterized** in that there is present on one end of the funnel an edge bead (4) so arranged as to snap into engagement in the flange part (24).

3. A funnel according to Patent Claim 1, **characterized** in that the bead (7) arranged on the inside of the funnel is angled in relation to the line of the edge of the funnel.

4. A funnel according to Patent Claims 1 and 2, **characterized** in that the funnel is provided at its larger end (11) with an edge part (2) extending around the opening exhibiting a straight cylindrical generating surface (33) and a conical generating surface (34) set at an angle to the line of said straight cylindrical generating surface and so arranged as to engage by means of a snap-in lock around the flange part (24) of the first head (6).

## Patentansprüche

1. Trichter zur Anbringung an medizinischen Instrumenten zur Diagnostik und Behandlung von Öffnungen von Körperkanälen, der mit einem Kopf (6,9) von wenigstens zwei unterschiedlichen Arten von Instrumenten verbindbar ist, von denen ein Kopf (6) ein ringförmiges Flanschteil (24) und der andere Kopf (9) ein trichterförmiges Teil (12) mit einer Nut (8) aufweist, wobei der Trichter eine kleine Vorderöffnung (10) und eine größere rückwärtige Öffnung (11) zusammen mit einer durchlaufenden Öffnung zwischen beiden aufweist, und wobei der Trichter an seinem größeren Ende (11) mit einer Kante (2) versehen ist, die sich um die Öffnung erstreckt und so angeordnet ist, daß sie mittels eines Einschnappverschlusses mit dem Flansch (24) des ersten Kopfes (6) in Eingriff kommt und das die Innenseite des Trichters mit einer Wulst (7) versehen ist, die so angeordnet ist, daß sie in eine Nut (8) am zweiten Kopf (9) angreift, wobei der Trichter in einer einzigen Ausführung zur Benutzung mit beiden vorher erwähnten Kopfarten geeignet ist.

2. Trichter nach Anspruch 1, dadruch gekennzeichnet, daß an einem Ende des Trichters eine Kantenwulst (4) so angeordnet ist, daß sie in den Flanschteil (4) einschnappt.

3. Trichter nach Anspruch 1 dadurch gekennzeichnet, daß die Wulst (7) an der Innenseite des Trichters unter einem Winkel bezogen auf die Kantenlinie des Trichters angeordnet ist.

4. Trichter nach Anspruch 1 und 2 dadurch gekennzeichnet, daß der Trichter an seinem größeren Ende (11) mit einer Kante (2) versehen ist, die sich um die Öffnung erstreckt, die eine gerade zylinderische Oberfläche (33) zeigt und eine konische Fläche (34) mit einem Winkel zur linie der geraden zylindrischen Oberfläche und so angeordnet ist, daß sie mittels eines Schnappverschlusses um den Flansch (24) des ersten Kopfes (6) in Eingriff bringbar ist.

## Revendications

1. Entonnoir de fixation d'instruments médicaux utilisés pour le diagnostic et le traitement des orifices de canaux physiologiques, conçu de façon à être fixable à une tête (6, 9) de l'instrument d'au moins deux types différents, une tête (6) présentant une partie à bride annulaire (24) et l'autre tête (9) présentant une partie en forme d'entonnoir (12) munie d'une rainure (8), lequel entonnoir présente une petite ouverture frontale (10) et une grande ouverture arrière (11), ainsi qu'un trou traversant entre elles, dans lequel l'entonnoir est muni à sa grande extrémité (11) d'une partie bord (2) s'étendant autour de l'ouverture, disposée de façon à s'enfiler au moyen d'un verrou à ressort autour de la partie à bride (24) sur la première tête (6), et dans lequel l'intérieur de l'entonnoir est muni d'un galet (7) disposé de façon à s'insérer dans la rainure (8) de la seconde tête (9), l'entonnoir étant utilisable, selon une seule conception, pour les deux types de tête susmentionnés.

2. Entonnoir selon la revendication 1, caractérisé en ce qu'est présente sur une extrémité de l'entonnoir un galet de bord (4) disposé de façon à s'enfoncer par déclic dans la partie à bride (24).

3. Entonnoir selon la revendication 1, caractérisé en ce que le galet (7) disposé à l'intérieur de l'entonnoir forme un angle par rapport à la ligne du bord de l'entonnoir.

4. Entonnoir selon les revendications 1 et 2, caractérisé en ce que l'entonnoir est muni à sa grande extrémité (11) d'une partie bord (2) s'étendant autour de l'ouverture présentant une surface génératrice cylindrique droite (33) et une surface génératrice conique (34) formant un angle avec la ligne de ladite surface génératrice cylindrique droite et disposée de façon à s'enfiler au moyen d'un verrou à ressort sur la partie à bride (24) de la première tête (6).
